# EUROPEAN PATENT APPLICATION

(11) **EP 4 230 636 A1**
(43) Date of publication of application: **23.08.2023**
(21) Application number: 21909315.0
(22) Date of filing: 20.12.2021
(51) Int. Cl.: C07H 15/203, C07H 1/00

(54) **METHOD FOR AUTOMATIC PREPARATION OF FONDAPARINUX SODIUM PENTOSACCHARIDE INTERMEDIATE**

(30) Priority: 21.12.2020 CN 202011518943
(71) Applicant: Peking University, Beijing 100871 (CN)
(72) Inventor: YE, Xinshan, Beijing 100871 (CN); YAO, Wenlong, Beijing 100871 (CN); XIONG, Decai, Beijing 100871 (CN)
(74) Representative: FRKelly
(86) International application number: PCT/CN2021/139518
(87) International publication number: WO 2022/135319

(57) **Abstract**

An automatic preparation method of a fondaparinux sodium pentosaccharide intermediate is providedvia an automatic preparation device. In the preparation method, the automatic preparation of three components (D+EF+GH) is realized through automatic sampling and monitoring, and a fully-protected fondaparinux sodium pentosaccharide intermediate (shown in formula I) is obtained. In this way, automatic synthesis of the fondaparinux sodium pentosaccharide intermediate is realized, which saves manpower and improves efficiency and productivity, and has high safety and reproducibility. The preparation method can be directly monitored online, which is convenient for optimizing and monitoring a real-time status of reactions. Furthermore, automatic temperature control can better meet the needs of the reactions for temperature rise and fall. The preparation method adopts a "pre-activation" one-pot mode, which reduces the number of separations and is easy to operate. Moreover, the method selects commonly-used ester protecting groups, has higher stereoselectivity and yield, and can use general-purpose deprotection measures. Therefore, the present disclosure has a great significance for reducing a production cost and achieving large-scale production of fondaparinux sodium. The definition of each substituent in the formula I is the same as the definition in the description.

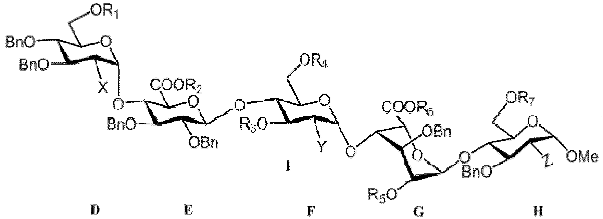

## Description

### TECHNICAL FIELD

The present disclosure relates to the technical field of medicine, in particular to an automatic preparation method of a fondaparinux sodium pentosaccharide intermediate.

### BACKGROUND

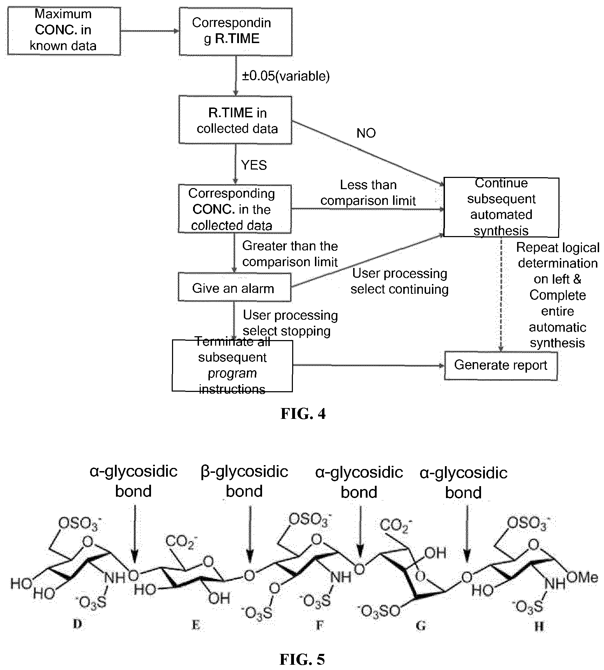

Heparin and heparin sulfate are a highly important class of linear sulfated polysaccharides in glycosaminoglycans. Heparin and heparin sulfate are found on the surface of most animal cells, and play key roles in various physiological processes, such as anticoagulation, antiviral infection, inflammatory response, cell adhesion, cell growth regulation, lipid metabolism, and tumor metastasis. Fondaparinux sodium (Arixtra) is the minimum pentosaccharide domain that can specifically bind to antithrombin III, and has a structural formula shown in FIG. 5. The five saccharide rings of the fondaparinux sodium are named D, E, F, G, and H rings according to customary nomenclature.

Fondaparinux sodium is the first and currently the only synthetic heparin oligosaccharide-based anticoagulant that has been marketed. This drug was launched in 2002, with a trade name of "Arixtra". The fondaparinux sodium is widely used in lower extremity orthopedic surgery to prevent the occurrence of venous thrombosis, and has global market sales of approximately US$190 million in 2017. Compared with other low-molecular heparins, the fondaparinux sodium has a significant anticoagulant activity, less side effects, and a longer half-life. However, artificial synthesis with more than 50 steps and a total yield of less than 0.1% makes fondaparinux sodium the most expensive heparin drug, which greatly limits its wide application. Since the patent expired in 2008, there have been numerous (3+2)[Chem. Med. Chem 2014, 9, 1071-1080; J. Org. Chem., 2016, 81, 162-184.] and (4+1)[Angew. Chem. Int. Ed., 2014, 53, 9876-9879.] convergent synthesis methods, a one-pot three-component synthesis method *(*Org. Chem. Front., 2019, 6, 3116-3120; Org. Lett., 2020, 22, 4638-4642.), enzymatic and chemoenzymatic extended synthesis methods, and a sulfated modification method (Science, 2011, 334, 498-501.) for fondaparinux sodium pentosaccharide. However, these methods all require highly specialized professionals with special training in glycochemistry to be completed in special glycochemistry laboratories, and are time-consuming and laborious. This substantially increases a cost of fondaparinux sodium acquisition. Therefore, there is still an urgent need for better strategies and technologies to improve an acquisition efficiency of the fondaparinux sodium pentosaccharide. If a fully-protected fondaparinux sodium pentosaccharide intermediate can be successfully obtained efficiently and quickly by automatic synthesis, the cost of fondaparinux sodium acquisition can be significantly reduced. This is of great significance for the large-scale production and application of fondaparinux sodium.

### SUMMARY

An objective of the present disclosure is to provide an automatic preparation method of a fondaparinux sodium pentosaccharide intermediate. The preparation method can save the manpower, reduce the influence of human factors, and improve the acquisition efficiency of the fondaparinux sodium pentosaccharide intermediate.

To achieve the above objective, the present disclosure provides the following technical solutions:

The present disclosure provides an automatic preparation method of a fondaparinux sodium pentosaccharide intermediate, where the automatic preparation method is implemented by an automatic preparation device of the fondaparinux sodium pentosaccharide intermediate; the automatic preparation device includes: a reactor, an upper computer, an inert gas device, a first activator container, a second activator container, a first sample container, a second sample container, a third sample container, an automatic sample injection system, a low-temperature circulating device, and a magnetic stirring device;

the inert gas device is connected with the automatic sample injection system, the first activator container, the second activator container, the first sample container, the second sample container, and the third sample container respectively; the automatic sample injection system is connected with the reactor; and the automatic sample injection system, the low-temperature circulating device, and the magnetic stirring device each are connected with the upper computer;

the fondaparinux sodium pentosaccharide intermediate has a structure shown in formula I, and is named DEFGH-1; 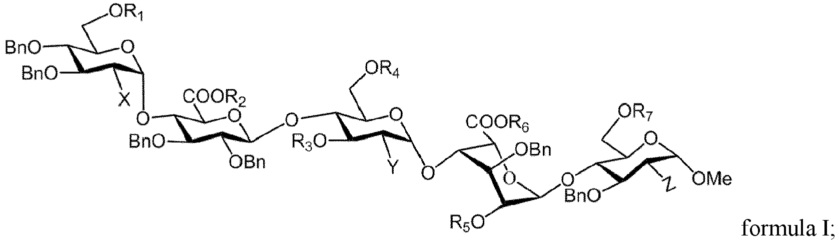
in the formula I, R₁ is selected from the group consisting of an acyl group and a silicon protecting group; R₂ and R₆ are independently carboxyl protecting groups; R₃ and R₄ are independently acyl groups; R₅ and R₇ are independently acyl groups; and X, Y, and Z are independently selected from the group consisting of N₃ and various amino groups with a protecting group;
the fondaparinux sodium pentosaccharide intermediate is prepared from a compound D-1, a compound EF-1, and a compound GH-1; and
the automatic preparation method includes the following steps:
adding the compound D-1 into the first sample container; where the compound D-1 has a structure shown in formula II: 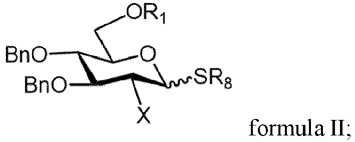
in the formula II, R₁ is selected from the group consisting of an acyl group and a silicon protecting group; SRs is a glucosinolate leaving group; and X is selected from the group consisting of N₃ and various amino groups with a protecting group;
adding the compound EF-1 into the second sample container; where the compound EF-1 has a structure shown in formula III: 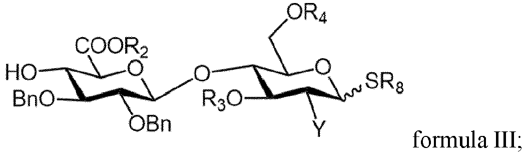
in the formula III, R₂ is a carboxyl protecting group, and R₃ and R₄ are independently acyl groups; SRs is a glucosinolate leaving group; and Y is selected from the group consisting of N₃ and various amino groups with a protecting group;
adding the compound GH-1 into the third sample container; where the compound GH-1 has a structure shown in formula IV: 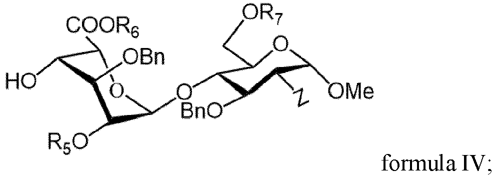
in the formula IV, R₅ and R₇ are independently acyl groups, R₆ is a carboxyl protecting group, and Z is selected from the group consisting of N₃ and various amino groups with a protecting group;
gas protection: filling the first sample container, the second sample container, the third sample container, the first activator container, the second activator container, and the reactor with an inert gas by the automatic sample injection system;
cooling the reactor by the low-temperature circulating device;
delivering the compound D-1 to the reactor by the automatic sample injection system;
stirring the compound D-1 in the reactor by the magnetic stirring device, and conducting pre-drying;
delivering a first activator in the first activator container and a second activator in the second activator container to the reactor sequentially by the automatic sample injection system, and conducting pre-activation I on the compound D-1 for a first set time;
delivering the compound EF-1 to the reactor by the automatic sample injection system;
subjecting the reactor to programmed heating by the low-temperature circulating device, and conducting a reaction I for a second set time;
cooling the reactor by the low-temperature circulating device;
delivering the first activator in the first activator container and the second activator in the second activator container to the reactor sequentially by the automatic sample injection system, and conducting pre-activation II on an intermediate obtained by a reaction of the compound D-1 and the compound EF-1 for a third set time;
delivering the compound GH-1 to the reactor by the automatic sample injection system;
subjecting the reactor to programmed heating by the low-temperature circulating device, and conducting a reaction II for a fourth set time; and
delivering a quenching reaction solvent to the reactor by the automatic sample injection system, and terminating the reaction II to obtain a compound DEFGH-1.

According to specific examples provided by the present disclosure, the present disclosure discloses the following technical effects:
The present disclosure provides an automatic preparation method of a fondaparinux sodium pentosaccharide intermediate. In the present disclosure, the automatic sample injection system is controlled by the upper computer to conduct automatic sampling, and the detection device is controlled by the upper computer to conduct automatic on-line detection on reactants in the reactor. In this way, the automatic preparation of the fondaparinux sodium pentosaccharide intermediate is realized, with saved manpower and reduced influence of human factors. Therefore, an acquisition efficiency of the fondaparinux sodium pentosaccharide intermediate is improved, and the cost of the fondaparinux sodium pentosaccharide intermediate is reduced.

### BRIEF DESCRIPTION OF THE DRAWINGS

To describe technical solutions in embodiments of the present disclosure or in the prior art more clearly, the accompanying drawings required for the embodiments are briefly described below. Apparently, the accompanying drawings in the following description show merely some embodiments of the present disclosure, and those of ordinary skill in the art can still derive other accompanying drawings from these accompanying drawings without creative efforts.
FIG. 1 shows a structural representation of the automatic preparation device of a fondaparinux sodium pentosaccharide intermediate of the present disclosure;
FIG. 2 shows a schematic diagram of the detection device of the present disclosure;
FIG. 3 shows a schematic flow chart of the automatic preparation method of a fondaparinux sodium pentosaccharide intermediate of the present disclosure;
FIG. 4 shows a schematic flow chart of comparison and determination of the detection device of the present disclosure;
FIG. 5 shows a schematic diagram of a structural formula of the pentosaccharide of the present disclosure;
FIG. 6 shows a schematic diagram of a reaction of the compound D-1, the compound EF-1, and the compound GH-1 to generate DEFGH-1 of the present disclosure;
FIG. 7 shows an online monitoring interface of the upper computer of the present disclosure;
FIG. 8 shows a schematic diagram of a preparation principle of the compound D-1 of the present disclosure;
FIG. 9 shows a schematic diagram of a preparation principle of the compound EF-1 of the present disclosure; and
FIG. 10 shows a schematic diagram of a reaction of a compound D-3, a compound EF-4, and a compound GH-2 to generate DEFGH-2 in an example of the present disclosure.

### Reference Numerals:

1. reactor, 2. inert gas device, 3. first sample container, 4. second sample container, 5. third sample container, 6-1. first activator container, 6-2, second activator container, 7. first switching valve, 8. second switching valve, 9. first syringe pump, 10. second syringe pump, 11. magnetic stirring device, 12. low-temperature circulating device, 13. waste gas treatment device, 14. waste liquid treatment device, 15. first solenoid valve, 16. second solenoid valve, 17. third solenoid valve, 18. fourth solenoid valve, 19. fifth solenoid valve, 20. pressure sensor, 21. flow meter, 30. sampling device, 31. power module, 32. monitoring and analysis module, and 33. upper computer.

### DETAILED DESCRIPTION OF THE EMBODIMENTS

The technical solutions of the embodiments of the present disclosure are clearly and completely described below with reference to the accompanying drawings. Apparently, the described embodiments are merely a part rather than all of the embodiments of the present disclosure. All other embodiments obtained by those of ordinary skill in the art based on the embodiments of the present disclosure without creative efforts shall fall within the protection scope of the present disclosure.

An objective of the present disclosure is to provide an automatic preparation method of a fondaparinux sodium pentosaccharide intermediate. The preparation method can save the manpower, reduce the influence of human factors, improve the acquisition efficiency of the fondaparinux sodium pentosaccharide intermediate, and lower the cost.

To make the above objectives, features, and advantages of the present disclosure clearer and more comprehensible, the present disclosure will be further described in detail below with reference to the accompanying drawings and the specific examples.

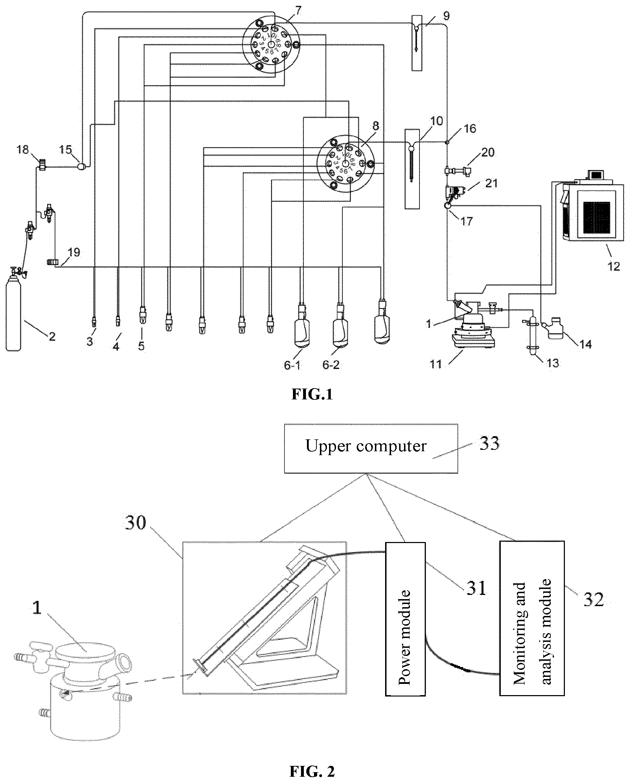

FIG. 1 shows a structural representation of the automatic preparation device of a fondaparinux sodium pentosaccharide intermediate of the present disclosure; and FIG. 2 shows a schematic diagram of the detection device of the present disclosure. As shown in FIG. 1 to FIG. 2, the present disclosure discloses an automatic preparation device of a fondaparinux sodium pentosaccharide intermediate. The automatic preparation device includes: a reactor 1, an upper computer 33, an inert gas device 2, a first activator container 6-1, a first activator container 6-2, a first sample container 3, a second sample container 4, a third sample container 5, switching valves, syringe pumps, a low-temperature circulating device 12, a magnetic stirring device 11, and a detection device. The automatic sample injection system includes the switching valves and the syringe pumps.

The inert gas device 2 is connected to the switching valves, the first activator container 6-1, the second activator container 6-2, the first sample container 3, the second sample container 4, and the third sample container 5 respectively. The first sample container 3, the second sample container 4, the third sample container 5, the first activator container 6-1, and the second activator container 6-2 are connected to the switch valves. The switching valves are connected to the reactor 1 through the syringe pumps respectively. The detection device is connected to the reactor 1. The switching valves, the syringe pumps, the low-temperature circulating device 12, the magnetic stirring device 11, and the detection device are connected to the upper computer 33. The low-temperature circulating device 12 provides a low-temperature environment for the reactor 1, and the magnetic stirring device 11 is used to stir reactants in the reactor 1.

The switching valves include a first switching valve 7 and a second switching valve 8. The syringe pumps include a first syringe pump 9 and a second syringe pump 10. A measuring range of the second syringe pump 10 is larger than that of the first syringe pump 9. The first switching valve 7 is connected to the first syringe pump 9, and the second switching valve 8 is connected to the second syringe pump 10.

The automatic preparation device further includes a flow meter 21, and the flow meter 21 is arranged on a pipeline between the syringe pumps and the reactor 1. The automatic preparation device further includes a pressure sensor 20, and the pressure sensor 20 is arranged on the pipeline between the syringe pumps and the reactor 1.

The automatic preparation device further includes a waste gas treatment device 13 and a waste liquid treatment device 14, both of which are connected to the reactor 1.

The inert gas device 2 is connected to a first port of the fourth solenoid valve 18 through a pressure regulating filter, and the inert gas device 2 is connected to a first port of the fifth solenoid valve 19 through a pressure regulating filter. A second port of the fourth solenoid valve 18 is connected to a first port of the first solenoid valve 15. A second port of the first solenoid valve 15 is connected to a tenth channel of the second switching valve 8, and a third port of the first solenoid valve 15 is connected to a tenth channel of the first switching valve 7. A second port of the fifth solenoid valve 19 is connected with the first sample container 3, the second sample container 4, the third sample container 5, the first activator container 6-1, and the second activator container 6-2. The first sample container 3 is connected to a first channel of the first switching valve 7, the second sample container 4 is connected to a second channel of the first switching valve 7, and the third sample container 5 is connected to a third channel of the first switching valve 7 respectively. The first activator container 6-1 is connected with a ninth channel of the first switching valve 7 and a ninth channel of the second switching valve 8 respectively, and the second activator container 6-2 is connected to an eighth channel of the first switching valve 7 and an eighth channel of the second switching valve 8 respectively.

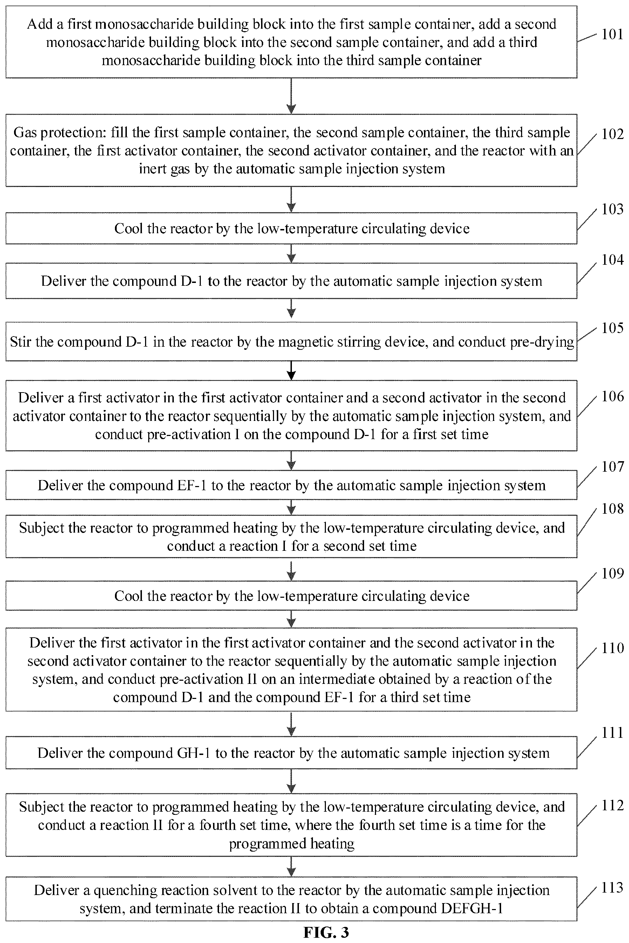

As shown in FIG. 3, the present disclosure further discloses an automatic preparation method of a fondaparinux sodium pentosaccharide intermediate. The fondaparinux sodium pentosaccharide intermediate has a structure shown in formula I, and is named DEFGH-1; 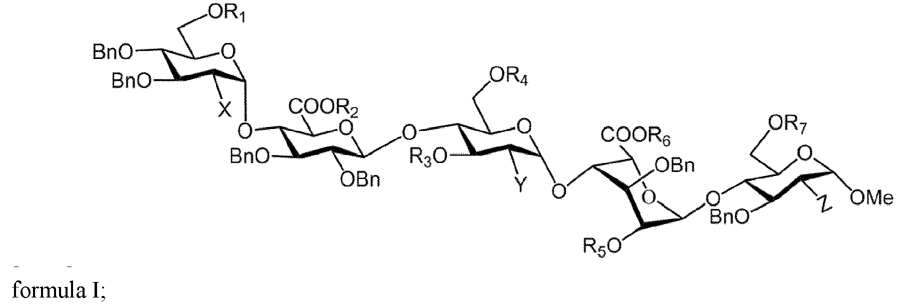
in the formula I, R₁ is selected from the group consisting of acyl group or a silicon protecting group, preferably acetyl (Ac) or *tert*-butyldiphenylsilyl (TBDPS); R₂ and R₆ are independently carboxyl protecting groups, preferably methyl (Me); R₃ and R₄ are independently acyl groups, preferably acetyl (Ac); R₅ and R₇ are independently acyl groups, preferably selected from the group consisting of acetyl (Ac) and benzoyl (Bz); X, Y, and Z are independently selected from the group consisting of N₃ or various amino groups with a protective group; and SRs is a glucosinolate leaving group, preferably, but not limited to, the group consisting of phenylthio, *p*-methylphenylthio, *o*-methylphenylthio, and 2-methyl-5-tert-butylphenylthio;
the fondaparinux sodium pentosaccharide intermediate is prepared from a compound D-1, a compound EF-1, and a compound GH-1; and
the automatic preparation method includes the following steps:
   Step 101: adding the compound D-1 into the first sample container 3; where the compound D-1 has a structure shown in formula II: 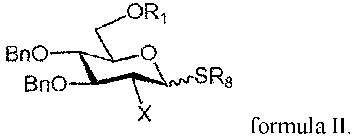
in the formula II, R₁ is selected from the group consisting of acyl group or a silicon protecting group, preferably acetyl (Ac) or *tert*-butyldiphenylsilyl (TBDPS); SRs is a glucosinolate leaving group, preferably, but not limited to, the group consisting of phenylthio, *p*-methylphenylthio, *o*-methylphenylthio, and 2-methyl-5-tert-butylphenylthio; and X is selected from the group consisting of N₃ and various amino groups with a protecting group. In the present disclosure, the compound D-1 specifically has a structure preferably shown in formula II-1, named a compound D-3: 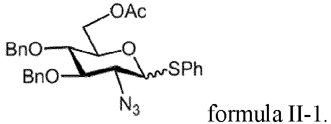
adding the compound EF-1 into the second sample container 4; where the compound EF-1 has a structure shown in formula III: 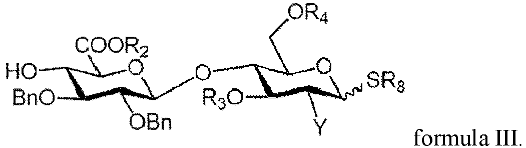
in the formula III, R₂ is a carboxyl protecting group, preferably methyl (Me); R₃ and R₄ are independently acyl groups, preferably acetyl (Ac); SRs is a glucosinolate leaving group, preferably, but not limited to, the group consisting of phenylthio, *p*-methylphenylthio, o-methylphenylthio, and 2-methyl-5-tert-butylphenylthio; and Y is selected from the group consisting of N₃ and various amino groups with a protecting group. In the present disclosure, the compound EF-1 specifically has a structure preferably shown in formula III-1, named a compound EF-4: 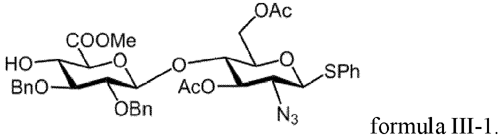
adding the compound GH-1 into the third sample container 5; where the compound GH-1 has a structure shown in formula IV: 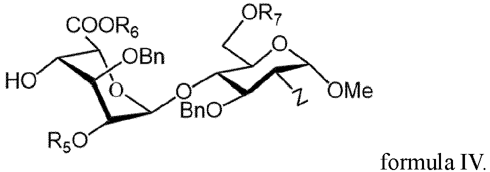
in the formula IV, R₅ and R₇ are independently acyl group, preferably selected from the group consisting of acetyl (Ac) and benzoyl (Bz); R₆ is a carboxyl protecting group, preferably methyl (Me); and Z is selected from the group consisting of N₃ and various amino groups with a protecting group. In the present disclosure, the compound GH-1 specifically has a structure preferably shown in formula IV-1, named a compound GH-2:

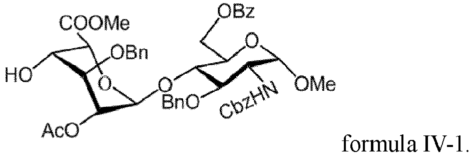

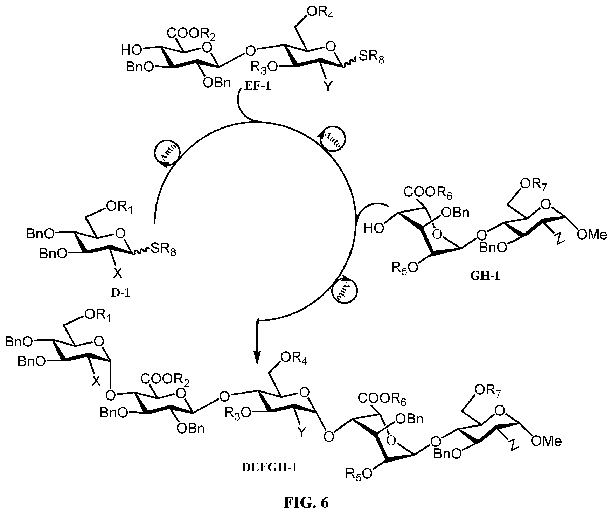

FIG. 6 shows a schematic diagram of a one-pot three-component reaction of the compound D-1, the compound EF-1, and the compound GH-1 to generate a fondaparinux sodium pentosaccharide intermediate, DEFGH-1.

When the compound D-1 has a structure preferably shown in formula II-1, the compound EF-1 has a structure preferably shown in formula III-1, and the compound GH-1 has a structure preferably shown in formula IV-1, an obtained fondaparinux sodium pentosaccharide intermediate has a structure shown in formula I-1, named a compound DEFGH-2:

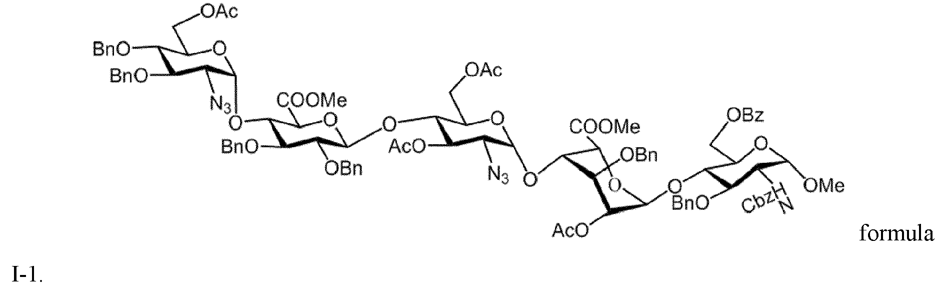

Step 102, gas protection: filling the first sample container, the second sample container, the third sample container, the first activator container, the second activator container, and the reactor with an inert gas by the automatic sample injection system.

Step 103: cooling the reactor by the low-temperature circulating device.

Step 104: delivering the compound D-1 to the reactor by the automatic sample injection system.

Step 105: stirring the compound D-1 in the reactor by the magnetic stirring device, and conducting pre-drying.

Step 106: delivering a first activator in the first activator container and a second activator in the second activator container to the reactor sequentially by the automatic sample injection system, and conducting pre-activation I on the compound D-1 for a first set time.

Step 107: delivering the compound EF-1 to the reactor by the automatic sample injection system.

Step 108: subjecting the reactor to programmed heating by the low-temperature circulating device, and conducting a reaction I for a second set time. The second set time is a time for the programmed heating.

Step 109: cooling the reactor by the low-temperature circulating device.

Step 110: delivering the first activator in the first activator container and the second activator in the second activator container to the reactor sequentially by the automatic sample injection system, and conducting pre-activation II on an intermediate obtained by a reaction of the compound D-1 and the compound EF-1 for a third set time.

Step 111: delivering the compound GH-1 to the reactor by the automatic sample injection system.

Step 112: subjecting the reactor to programmed heating by the low-temperature circulating device, and conducting a reaction II for a fourth set time. The fourth set time is a time for the programmed heating.

Step 113: delivering a quenching reaction solvent to the reactor by the automatic sample injection system, and terminating the reaction II to obtain a compound DEFGH-1.

The first activator is preferably *p*-Toluene sulfonyl chloride (*p*-TolSCl), and the second activator is preferably silver trifluoromethanesulfonate (AgOTf).

The reactor is cooled to -75°C by the low-temperature circulating device.

The magnetic stirring device has a rotational speed of 400 rpm to 1,000 rpm. The compound D-1 in the reactor is stirred for 1 min to 300 min by the magnetic stirring device.

The automatic preparation device further includes a detection device; and the detection device is connected to the reactor by means of a pipe, and is electrically connected to the upper computer.

The automatic preparation method of a fondaparinux sodium pentosaccharide intermediate further includes the following steps after the pre-activation I is conducted on the compound D-1 for the first set time: detecting whether there is remaining compound D-1 by the detection device, and conducting alarming if there is the remaining compound D-1. If there is no remaining, step 107 is conducted.

The automatic preparation method of a fondaparinux sodium pentosaccharide intermediate further includes the following steps after the pre-activation II is conducted on the intermediate obtained by the reaction of the compound D-1 and the compound EF-1 for the third set time:
detecting whether there is remaining intermediate obtained by the reaction of the compound D-1 and the compound EF-1 by the detection device, and conducting alarming if there is the remaining intermediate. If there is no remaining, step 111 is conducted.

The automatic preparation method of a fondaparinux sodium pentosaccharide intermediate further includes the following steps after the reactor is subjected to the programmed heating by the low-temperature circulating device, and the reaction II is conducted for the fourth set time: detecting whether there is remaining compound GH-1 by the detection device, and conducting alarming if there is the remaining compound GH-1. If there is no remaining, step 113 is conducted.

The quenching reaction solvent is triethylamine.

After step 113, the automatic preparation method further includes cleaning the pipeline, stopping refrigeration, stopping stirring, and stopping introducing the inert gas.

The detection device detects the compound D-1, the compound EF-1, or the compound GH-1 in the reactor 1 by high-performance liquid chromatography (HPLC).

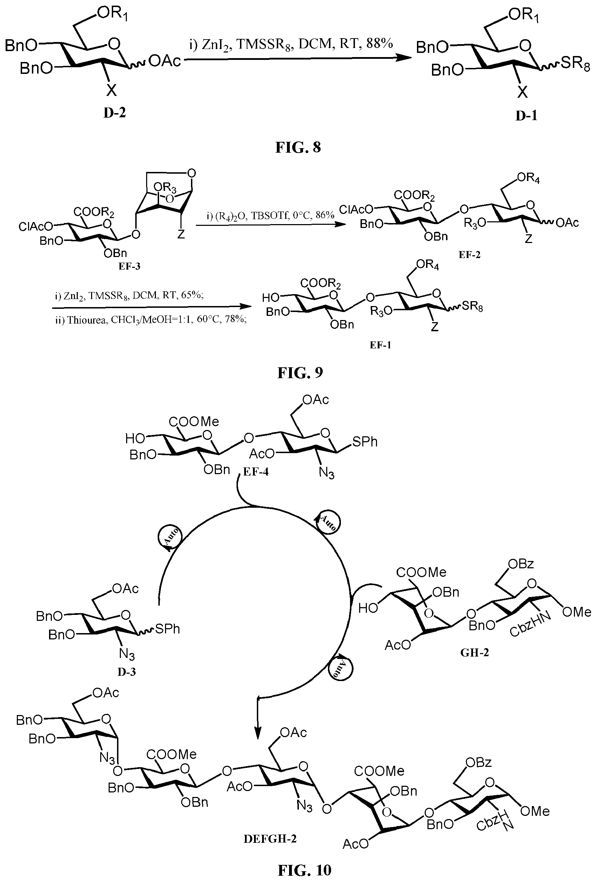

Referring to a literature report (Org. Chem. Front., 2019, 6, 3116-3120.), a preparation method of the D-1 includes: adding glucosinolate after acetylation of an anomeric position of commercial glucosamine D-2 A to obtain a stable monosaccharide building block D-1. The preparation method of D-1 is shown in FIG. 8.

Referring to the literature report (Org. Chem. Front., 2019, 6, 3116-3120.), a preparation method of the EF-1 includes: opening lactone from a commercial disaccharide intermediate EF-3 in the presence of *tert*-butyldimethylsilyltrifluoromethanesulfonate (TBSOTF) to obtain EF-4; adding the EF-4 with glucosinolate, and removing a temporary protecting group chloroacetyl under the action of thiourea to obtain the disaccharide intermediate EF-1. The preparation method of the EF-1 is shown in FIG. 9.

Referring to a literature report *(*Org. Chem. Front., 2019, 6, 3116-3120.), a general preparation method of a fully-protected pentosaccharide intermediate DEFGH-1 by deprotection and sulfonation includes: subjecting the DEFGH-1 to acyl group and carboxyl protecting group removal under strong alkali conditions, or to large-steric hindrance silicon protecting group removal under acidic conditions and then to the acyl groupand carboxyl protecting group removal under strong alkali conditions; conducting *O*-sulfonation under heating, and subjecting a resulting product to benzyl and amino protecting group removal by catalytic hydrogenation; reducing azide into amino, and conducting sulfonation on the amino to obtain the fondaparinux sodium (DEFGH).

In this example, the compound D-1, the compound EF-1, or the compound GH-1 in the reactor 1 are detected with the monitoring and analysis module 32 by HPLC.

The present disclosure further provides a computer-readable storage medium, which stores computer-executable instructions, where the computer-executable instructions are configured to execute the automatic preparation method of a fondaparinux sodium pentosaccharide intermediate.

The present disclosure discloses an automatic preparation method of a fondaparinux sodium pentosaccharide intermediate. Compared with the prior art, the present disclosure has the following advantages and beneficial effects: 1. In the present disclosure, the automatic preparation of the fondaparinux sodium pentosaccharide intermediate has been realized, and can save manpower, improve efficiency and productivity, and have higher safety and reproducibility. 2. The preparation method can be directly monitored online, which is convenient for optimizing and monitoring a real-time status of reactions. Furthermore, automatic temperature control can better meet the needs of the reactions for temperature rise and fall. 3. The preparation method adopts a "pre-activation" one-pot mode, which reduces the number of separations and is easy to operate. 4. Moreover, the method selects commonly-used ester protecting groups, has higher stereoselectivity and yield, and can use general-purpose deprotection conditions. Therefore, the present disclosure has a great significance for reducing a production cost and achieving large-scale production of fondaparinux sodium.

### Examples:

Before automation: *p*-TolSC1 (*p*-Toluene sulfonyl chloride) and AgOTf (silver trifluoromethanesulfonate) were preferred as a "pre-activation" system, and a stock solution of the *p*-TolSC1 was diluted in dichloromethane and connected to channel 6, a stock solution of the AgOTf was dissolved in a mixed solution of toluene and dichloromethane (V/V = 3:1) and connected to channel 5. However, other "pre-activation" system was available. Preferably, a freshly activated 4A molecular sieve was added to a reactor of a synthesizer in advance, and the reactor was sealed. The stock solutions (dissolved in a certain amount of dichloromethane) of the compound D-3 (connected to channel 1), the compound EF-4 (connected to channel 2), and the compound GH-2 (connected to channel 3) were prepared. The dry dichloromethane connected to channel 9 was used as a solvent for sampling or cleaning the pipeline. The quenching reaction solvent triethylamine was diluted in dichloromethane and connect to channel 8. Each switch of the synthesizer was turned on and a software control program was started, and an automatic synthesis program of the fondaparinux sodium pentosaccharide intermediate was written after automatic connection (Table 1). Specific instructions and parameters could be modified, deleted, added, sequenced, and inserted according to the response requirements. Most of the instructions had been modularized and could be inserted according to the requirements, which was simple and convenient.

During automation: the project was loaded after writing the program, the "Start" button was clicked, and the automatic preparation device automatically executed the written project program in sequence. The operator could pause and stop the automatic reaction process according to the actual situation.

**Table 1 Programs of automatic synthesis project of fondaparinux sodium pentosaccharide intermediate**

| Glyc osyla tion cycle | Se qu enc e | Instruction name | Parameter 1 | Parameter 2 | Parameter 3 | Parameter 4 | Parameter 5 | Note |
|---|---|---|---|---|---|---|---|---|
| | 1 | Gas protection | Time 1 (s) | Time 2 (s) | | | | Inert gas filling the system |
| | | | 20 | 20 | | | | |
| | 2 | Waiting for activation | Time (min) | | | | | |
| | | | 3 | | | | | |
| | 3 | Beginning cooling | Set temperature (°C) | Interval temperature (°C) | | | | |
| | | | 0 | 1 | | | | |
| | 4 | Turning off gas | No parameter | | | | | |
| | 5 | Beginning cooling | -50 | 3 | | | | |
| | 6 | Gas protection | 10 | 10 | | | | |
| | 7 | Waiting for activation | 1 | | | | | Cooling reactor (without turning off the gas) |
| | 8 | Turning off gas | | | | | | |
| | 9 | Beginning cooling | -78 | 5 | | | | |
| | 10 | Gas protection | 10 | 10 | | | | |
| | 11 | Waiting for activation | 1 | | | | | |
| | 12 | Transferring building blocks | Switching valve channel number | Flow rate (µL/s) | Injection volume (µL) | Compensati on volume (µE) | Filled volume (µL) | |
| | | | 1 | 80 | 5000 | 800 | 2200 | Auto-injecti on of D-3 (1.1 eq) and cleaning pipeline |
| I | 13 | Cleaning pipeline | Time 1 (s) | Switching valve channel number | Flow rate (µL/s) | Liquid-wash ing volume | Time 2 (s) | |
| | | | 10 | 9 | 80 | 5500 | 10 | |
| | 14 | Cleaning pipeline | 10 | 9 | 80 | 5500 | 10 | |
| | 15 | Turning off gas | | | | | | |
| | 16 | Starting stirring | Set rotational speed (rpm) | Delay time (min) | | | | Pre-drying |
| | | | 450 | 15 | | | | |
| | 17 | Sampling and monitoring | Sampling time (s) | Sampling position | Sampling speed (µL/s) | Sampling capacity (µL) | Dilution time (s) | Pre-storing **D-3** data |
| | | | 3 | 1 | 40 | 2800 | 30 | |
| | 18 | Waiting for activation | 1 | | | | | |
| | 19 | Gas protection | 10 | 10 | | | | Auto-injecti on of diluted *p*-TolSCl (1.15 eq) and AgOTf (3.3 eq) |
| | 20 | Waiting for activation | 1 | | | | | |
| | 21 | Injecting activator | Switching valve channel number | Flow rate (µL/s) | Injection volume (µL) | Compensati on volume ( µL) | Filled volume (µL) | |
| | | | 6 | 40 | 800 | 200 | 1000 | |
| | 22 | Cleaning pipeline | 10 | 9 | 40 | 1000 | 10 | |
| | 23 | Cleaning pipeline | 10 | 9 | 40 | 1000 | 10 | |
| | 24 | Injecting activator | Switching valve channel number | Flow rate (µL/s) | Injection volume (µL) | Compensati on volume ( µL) | Filled volume (µL) | |
| | | | 5 | 80 | 3000 | 500 | 2000 | |
| | 25 | Cleaning pipeline | 10 | 9 | 80 | 3500 | 10 | |
| | 26 | Cleaning pipeline | 10 | 9 | 80 | 3500 | 10 | |
| | 27 | Turning off gas | | | | | | |
| | 28 | Waiting for activation | 3 | | | | | |
| | 29 | Sampling and monitoring | 3 | 1 | 40 | 2800 | 30 | **D-3** disappearin 9 |
| | 30 | Monitoring result | Contrast limit | | | | | |
| | | | 5 | | | | | |
| | 31 | Gas protection | 10 | 10 | | | | Automatic injection of **EF-4** (1.0 eq) and pre-storing data |
| | 32 | Transferring building blocks | **2** | 40 | 800 | 200 | 1000 | |
| | 33 | Cleaning pipeline | 10 | 9 | 40 | 1000 | 10 | |
| | 34 | Cleaning pipeline | 10 | 9 | 40 | 1000 | 10 | |
| | 35 | Sampling and monitoring | 3 | 1 | 40 | 2800 | 30 | |
| | 36 | Turning off gas | | | | | | |
| | 37 | Reaction time | Set temperature (°C) | Interval temperature (°C) | Delay time (min) | | | |
| | | | -75 | 1 | 3 | | | |
| | 38 | Reaction time | -70 | 1 | 4 | | | |
| | 39 | Reaction time | -65 | 1 | 6 | | | |
| | 40 | Reaction time | -60 | 1 | 8 | | | |
| | 41 | Reaction time | -55 | 1 | 10 | | | |
| | 42 | Reaction time | -50 | 1 | 15 | | | |
| | 43 | Reaction time | -45 | 1 | 15 | | | |
| | 44 | Reaction time | -40 | 1 | 10 | | | |
| | 45 | Reaction time | -35 | 1 | 10 | | | After adding **EF-4,** programme d heating was conducted by gradient control (the temperature could be modified according to the actual needs of the reaction).12 9 min in total. |
| | 46 | Reaction time | -30 | 1 | 10 | | | |
| | 47 | Reaction time | -25 | 1 | 10 | | | |
| | 48 | Reaction time | -20 | 1 | 8 | | | |
| | 49 | Reaction time | -15 | 1 | 5 | | | |
| | 50 | Reaction time | -10 | 1 | 3 | | | |
| | 51 | Reaction time | -5 | 1 | 3 | | | |
| | 52 | Reaction time | 0 | 1 | 3 | | | |
| | 53 | Reaction time | 5 | 1 | 3 | | | |
| | 54 | Waiting for reaction | Time (min) | | | | | |
| | | | 1 | | | | | |
| | 55 | Gas protection | 20 | 20 | | | | **EF-4** disappeared , new peak appeared |
| | 56 | Sampling and monitoring | 4 | 1 | 40 | 2800 | 30 | |
| | 57 | Monitoring result | Contrast limit | | | | | |
| | | | 5 | | | | | |
| | 58 | Waiting for activation | 1 | | | | | |
| II | 59 | Beginning cooling | 0 | 1 | | | | Programme d cooling for next glycosylatio n |
| | 60 | Turning off gas | | | | | | |
| | 61 | Beginning cooling | -50 | 3 | | | | |
| | 62 | Gas protection | 10 | 10 | | | | |
| | 63 | Waiting for activation | 1 | | | | | |
| | 64 | Turning off gas | | | | | | |
| | 65 | Beginning cooling | -72 | 2 | | | | |
| | 66 | Gas protection | 10 | 10 | | | | |
| | 67 | Waiting for activation | 1 | | | | | |
| | 68 | Injecting activator | 6 | 40 | 800 | 200 | 850 | |
| | 69 | Cleaning pipeline | 10 | 9 | 40 | 1000 | 10 | |
| | 70 | Cleaning pipeline | 10 | 9 | 40 | 1000 | 10 | Auto-injecti on of diluted *p*-TolSCl (1.05 eq) and AgOTf (3.0 eq) |
| | 71 | Injecting activator | 5 | 80 | 2500 | 500 | 1500 | |
| | 72 | Cleaning pipeline | 10 | 9 | 80 | 3000 | 10 | |
| | 73 | Cleaning pipeline | 10 | 9 | 80 | 3000 | 10 | |
| | 74 | Turning off gas | | | | | | |
| | 75 | Waiting for activation | 3 | | | | | |
| | 76 | Sampling and monitoring | 4 | 1 | 40 | 2800 | 30 | New peak disappeared |
| | 77 | Monitoring result | 5 | | | | | |
| | 78 | Gas protection | 10 | 10 | | | | |
| | 79 | Transferring building blocks | 3 | 40 | 800 | 200 | 1000 | |
| | 80 | Cleaning pipeline | 10 | 9 | 40 | 1000 | 10 | |
| | 81 | Cleaning pipeline | 10 | 9 | 40 | 1000 | 10 | |
| | 82 | Sampling and monitoring | 4 | 1 | 40 | 2800 | 30 | |
| | 83 | Turning off gas | | | | | | |
| | 84 | Reaction time | -70 | 1 | 3 | | | |
| | 85 | Reaction time | -65 | 1 | 5 | | | |
| | 86 | Reaction time | -60 | 1 | 5 | | | |
| | 87 | Reaction time | -55 | 1 | 7 | | | |
| | 88 | Reaction time | -50 | 1 | 8 | | | |
| | 89 | Reaction time | -45 | 1 | 10 | | | Automatic injection of **GH-2** (0.9 eq) and pre-storing data, programme d heating to conduct reaction for 129 min in total. |
| | 90 | Reaction time | -40 | 1 | 15 | | | |
| | 91 | Reaction time | -35 | 1 | 15 | | | |
| | 92 | Reaction time | -30 | 1 | 15 | | | |
| | 93 | Reaction time | -25 | 1 | 10 | | | |
| | 94 | Reaction time | -20 | 1 | 10 | | | |
| | 95 | Reaction time | -15 | 1 | 8 | | | |
| | 96 | Reaction time | -10 | 1 | 7 | | | |
| | 97 | Reaction time | -5 | 1 | 5 | | | |
| | 98 | Reaction time | 0 | 1 | 3 | | | |
| | 99 | Reaction time | 5 | 1 | 3 | | | |
| | 10 0 | Waiting for reaction | 1 | | | | | **GH-2** disappeared , new peak appeared |
| | 10 1 | Gas protection | 20 | 20 | | | | |
| | 10 2 | Sampling and monitoring | 5 | 1 | 40 | 2800 | 30 | |
| | 10 3 | Monitoring result | 10 | | | | | |
| | 10 4 | Waiting for reaction | 1 | | | | | |
| | 10 5 | Injecting activator | **8** | 40 | 200 | 100 | 1000 | Quenching reaction |
| | 10 6 | Cleaning pipeline | 10 | 9 | 40 | 1000 | 10 | |
| | 10 7 | Cleaning pipeline | 10 | 9 | 40 | 1000 | 10 | |
| | 10 8 | Stopping refrigeration | No parameter | | | | | |
| | 10 9 | Stopping stirring | No parameter | | | | | |
| | 11 0 | Turning off gas | No parameter | | | | | |

Note: this table exemplified a general automation process for the fondaparinux sodium pentosaccharide intermediate with an amount of 0.08 mmol of EF-4 (1.0 eq). The specific parameters of each instruction changed with the feeding amount and the repeated use of the instructions. The specific parameters were listed when the instructions appeared for the first time, and were not listed during subsequent repeated occurrences. All instructions could be run alone, repeatedly, and independently according to a logical relationship to ensure the normal execution of automation.

Each instruction in Table 1 was explained in detail below.
1. Gas protection: a, the fourth solenoid valve 18 and the fifth solenoid valve 19 were opened, the first solenoid valve 15 communicated with the first switching valve 7, and the channel 10 of the first switching valve 7 communicated with the first syringe pump 9 (the first A switching valve 7-the first syringe pump 9). The second solenoid valve 16 and the third solenoid valve 17 were connected with a waste liquid bottle, the flow meter 21 was turned on, and the first syringe pump 9 conducted V-type ventilation for P1 sec (the gas source had a set pressure, such that the gas could pass through smoothly), to empty the air and other gases in the pipeline between the first switching valve 7 and the waste liquid. b, the first solenoid valve 15 communicated with the second switching valve 8, and the channel 10 of the second switching valve 8 communicated with the second syringe pump 10 (the second switching valve 8-the second syringe pump 10). The second syringe pump 10 conducted V-type ventilation for P2 sec, to empty the air and other gases in the pipeline between the second switching valve 8 and the waste liquid. c, the third solenoid valve 17 was connected with a reaction bottle. The second syringe pump 10 conducted V-type ventilation and ran all the time to empty the air and other gases in the pipeline between the second switching valve 8 and the reactor 1 (to ensure that all pipelines were filled with argon, the flow meter 21 was opened during the whole process), until there was an instruction to close the gas path. Otherwise, there was always gas being discharged to atmosphere (atmospheric pressure) passing through the path "fourth solenoid valve 18-first solenoid valve 15-second switching valve 8-second syringe pump 10-second solenoid valve 16-third solenoid valve 17-reactor 1-gas". The V-type operation of the syringe pump meant that a sample inlet pipeline and a sample outlet pipeline of the syringe pump were directly connected, and the gas did not go through the suction and discharge processes of the syringes.
   Control parameters: the P1 was a V-type operation time (s) of the first syringe pump 9, and the P2 was a V-type operation time (s) of the second syringe pump 10.
2. Starting cooling: the ultra-low-temperature circulating device 12 was controlled to start cooling and circulation according to the set temperature until an actual temperature reached a range of the set temperature (P1') ± interval temperature (P2'). The set temperature was maintained until new instructions were received. Control parameters: the P1' was the set temperature (°C), and the P2' was the interval temperature (°C).
3. Turning off gas: the fourth solenoid valve 18 and the fifth solenoid valve 19 were closed. There was no control parameter.
4. Transferring building blocks: before running this instruction, the gas protection instruction should be run. The PLC determined which syringe pump the first solenoid valve 15 communicated with according to a sum of an injection volume P3 and a compensation volume P4: when the volume sum was greater than a range of the syringe pump with a small range, the first solenoid valve 15 communicated with the second syringe pump 10 with a large range; when the volume sum was smaller than the range of the syringe pump with a small volume, the first solenoid valve 15 communicated with the first syringe pump 9 with a small volume. When the second syringe pump 10 communicated with the corresponding second switching valve 8, different samples were drawn according to the channel number A1 of the second switching valve 8. The second solenoid valve 16 and the third solenoid valve 17 communicated with the waste liquid, and the second switching valve 8 changed from channel 10 to channel A1. The flow meter 21 was turnedon, the second syringe pump 10 pumped for a filled volume P5 (µL) at a flow rate of A2 µL/s, and then discharged to the waste liquid pipeline, such that the pipelines from the second syringe pump 10 to the third solenoid valve 17 were filled with sample. The second syringe pump 10 pumped the sum of the injection volume P3 and the compensation volume P4 from the channel A1 at a flow rate of A2 µL/s. When the sampling was just completed, the fifth solenoid valve 19 was closed, the flow meter 21 was reset, and the third solenoid valve 17 was switched to the reactor 1. The flow meter 21 started counting and controlled to switch the third solenoid valve 17 to communicate with the waste liquid when the desired injection volume P3 was reached, until the syringe pumps were completely discharged. If the syringe pumps were completely discharged and the desired injection volume was not reached, there might be no sample solution in the storage bottle or the sample solution was not enough, and an alarm was given. Control parameters: the A1 was a channel number of the second switching valve, and the A2 was the flow rate of the syringe pump; the P3 was the injection volume; the P4 was the compensation volume; and the P5 was the filled volume.
5. Cleaning pipeline: this instruction was generally executed after the transferring building blocks and the injecting activator, or could be executed alone.
   5.1. Air flushing pipeline to waste liquid (air flushing pipeline): the fourth solenoid valve 18 and the fifth solenoid valve 19 were opened, the second solenoid valve 16 and the third solenoid valve 17 were connected to the waste liquid, the first solenoid valve 15 communicated with the second switching valve 8, and the second switching valve 8 returned to channel 10 and communicated with the second syringe pump 10. The second syringe pump 10 conducted V-type operation for B1 sec (the gas flowed from a path of "fourth solenoid valve 18-first solenoid valve 15-first switching valve 7-first syringe pump 9-second solenoid valve 16-third solenoid valve 17"), to flush the pipelines between the second switching valve 8 and the third solenoid valve 17 to waste liquid by blowing gas. Control parameters: the B1 was a V-type operation time (s) of the second syringe pump 10.
   5.2. Solvent flushing pipeline to waste liquid (liquid washing pipeline): the second switching valve 8 was switched from channel 10 to channel A1 (the solvent was generally dichloromethane), and communicated with the second syringe pump 10. The second syringe pump 10 pumps C4 µL of the dichloromethane at a flow rate of C3 µL/s and discharged to clean the pipeline from the second switching valve 8 to the waste liquid, and returned to its original position. Control parameters: the A1 was a channel number of the second switching valve, the C3 was the flow rate of the first syringe pump (µL/s), and the C4 was a liquid washing volume (µL).
   5.3. Air flushing pipeline to waste liquid (air flushing pipeline): the second switching valve 8 was returned from the channel P2to the channel 10 and communicated with the second syringe pump 10. The first syringe pump 9 conducted V-type operation for B5 (s), to flush the pipeline (containing the cleaning solvent dichloromethane) between the second switching valve 8 and the third solenoid valve 17 to the waste liquid by blowing gas, and returned to its original position. Control parameters: the B5 was a V-type operation time (s) of the first syringe pump 9.
   5.4. Air flushing pipeline to reactor 1 (air flushing pipeline): after operating for B5 (s), the third solenoid valve 17 was switched from the waste liquid to communicate with the reactor 1 to flush the pipeline between the third solenoid valve 17 and the reactor 1 to the reactor 1 by blowing gas. Moreover, this solenoid valve was operating to keep the gas being always discharged through a venting port of reactor 1 until a new instruction was issued (at this time, the first solenoid valve 15, the second solenoid valve 16, the third solenoid valve 17, the fourth solenoid valve 18, and the fifth solenoid valve 19 were all open, and communicated with the reactor 1 through the second switching valve 8 and the second syringe pump 10). Control parameters: the parameters of B5 were adopted.
6. Starting stirring: a magnetic stirring function of the magnetic stirring device 11 was set by a visual control interface of a digital display. A rotational speed was set to D1 (rpm) to start stirring for a delay time of D2 (min). That is, the timing started after reaching the set speed, and the instruction ended after the set delay time was reached, and the next instruction could be executed. Control parameters: the D1 was the rotational speed, and the D2 was the delay time (min).
7. Injecting activator: this instruction was similar to the action of transferring building blocks, except that various activators were connected to the channels of the switching valves. Before running this instruction, the gas protection instruction should be run. The PLC determined which syringe pump the first solenoid valve 15 communicated with according to a sum of an injection volume P3 and a compensation volume P4: when the volume sum was greater than a range of the syringe pump with a small range, the first solenoid valve 15 communicated with the second syringe pump 10 with a large range; when the volume sum was smaller than the range of the syringe pump with a small volume, the first solenoid valve 15 communicated with the first syringe pump 9 with a small volume. When the second syringe pump 10 communicated with the corresponding second switching valve 8, different samples were drawn according to the channel number A1 of the second switching valve 8. The second solenoid valve 16 and third solenoid valve 17 communicated with the waste liquid, and the second switching valve 8 changed from channel 10 to channel A1. The flow meter 21 was turnedon, the second syringe pump 10 pumped for a filled volume P5 (µL) at a flow rate of A2 µL/s, and then discharged to the waste liquid pipeline, such that the pipelines from the second syringe pump 10 to the third solenoid valve 17 were filled with sample. The second syringe pump 10 pumped the sum of the injection volume P3 and the compensation volume P4 from the channel A1 at a flow rate of A2 µL/s. When the sampling was just completed, the fifth solenoid valve 19 was closed, the flow meter 21 was reset, and the third solenoid valve 17 was switched to the reactor 1. The flow meter 21 started counting and controlled to switch the third solenoid valve 17 to communicate with the waste liquid when the desired injection volume P3 was reached, until the syringe pumps were completely discharged. If the syringe pumps were completely discharged and the desired injection volume was not reached, there might be no sample solution in the storage bottle or the sample solution was not enough, and an alarm was given. Control parameters: the A1 was a channel number of the second switching valve, and the A2 was the flow rate of the second syringe pump; the P3 was the second injection volume; the P4 was the compensation volume; and the P5 was the filled volume.
8. Waiting for activation: this instruction was a waiting time for activating raw materials after the activator was injected, which was a "pre-activation" time, such that the instruction was called the "waiting for activation". A set parameter was generally E1 (min), and could also be used to replace the instruction of waiting time, which could be used repeatedly. Control parameter: the E1 was the waiting time for activation (min).
9. Reaction time: this instruction mainly controlled the ultra-low-temperature circulating device 12 to meet the demand changes of reaction temperature and time, which was different from the starting cooling. The ultra-low-temperature circulating device 12 changed the temperature and started to operate according to a set temperature F1 until an actual temperature reached a range of the set temperature F1 ± an interval temperature F2. Timing was started at this time, and the next instruction was executed after a delay time F3 was met. A purpose of programmed heating and cooling was achieved by repeatedly using the reaction time instruction. Control parameters: the F1 was the set temperature (°C), the F2 was the interval temperature (°C), and the F3 was the delay time (min).
10. Waiting for reaction: this instruction was a waiting time for sampling and monitoring after the reaction was basically completed, and was different from the waiting for activation. A set parameter was generally G1 (min), and could also be used to replace the instruction of waiting time, which could be used repeatedly. Control parameters: the G1 was a waiting time for sampling and monitoring (min).
11. Stopping stirring: the magnetic stirring device was controlled to stop stirring. There was no control parameter.
12. Stopping refrigeration: the low-temperature circulating device 12 was controlled to stop the refrigeration and circulation.
   Overall analysis: the entire automation process had the highest requirements on the accuracy of sample injection. Therefore, cross-contamination was avoided by dual control of the syringe pumps and the flow meter 21 with appropriate instructions to flush the pipelines. In this way, accurate sampling was achieved while ensuring the stable operation of the overall system.
13. Sampling and monitoring: this was generally executed after the instruction "waiting for activation" of the automatic preparation device.

Sampling and monitoring: the main functions of this instruction were automatic sampling, sample injection, pipeline cleaning, and triggering of HPLC operation. The detection device was shown in FIG. 2. The third syringe pump operated forward at a sampling speed (H3, µL/s) and drew a reaction solution to a certain volume, which was a sampling volume (H4, µL). At the beginning, air entered the syringe of the third syringe pump, and a slide rail drove a stainless steel needle down to a certain sampling position (H2, divided into 1, 2, and 3 gears), and punctured an airtight rubber gasket of the sampling port. The needle was in contact with the reaction solution for a residence time of the sampling time (H1, s, generally the time was very short). The slide rail went up to draw out the reactor 1 (the third syringe pump kept running such that the reaction solution did not drip), and a mechanical turntable rotated to a certain angle and stopped. The slide rail went down to puncture a bottle mouth of an organic solvent (usually acetonitrile) storage bottle, and the needle was in contact with the organic solvent for a residence time for diluting the reaction solution, which was a dilution time (H5, s, which was much longer than the reaction solution contact time, and the slide rail could stop for a certain period of time). At this time, the organic solvent could dilute the reaction solution and entered the syringe of the third syringe pump together. The top of the syringe was filled with air, and the syringe acted as a storage vial for a diluted reaction solution. The slide rail went up to draw out the organic solvent storage bottle, and the mechanical turntable continued to rotate until the waste liquid bottle went down for a certain distance (without puncturing) to wait for reverse cleaning to the waste liquid. The third syringe pump continued to operate in a positive direction, which was equivalent to diluting and mixing. The syringe sucked down for a certain distance and reached a set volume (X µL) and then discharged the diluted reaction solution upwards. The solenoid valve was connected to a high-pressure flow path switching valve, and the high-pressure flow path switching valve was in a sampling state, and the samples were injected by a power of the third syringe pump until the third syringe pump returned to its original position. The diluted reaction solution was injected into a loop (quantitative loop) of the high-pressure flow path switching valve. After sample injection, the software of the upper computer gave a short-circuit signal similar to a switch signal, to trigger HPLC to start running. The HPLC controlled the high-pressure flow path switching valve to change to a sample injection state, and after waiting for a certain period of time, it returned to the sampling state for the next sampling. After the HPLC operated for 3 min to 8 min, a TXT report was given immediately, and then output to a designated folder of the upper computer. After the HPLC operated, the column was automatically balanced for the next sampling and monitoring. While the HPLC was running and analyzing, the solenoid valve was connected to the organic solvent storage bottle (as a middle bottle), and the third syringe pump reversely absorbed a same volume of the organic solvent (usually acetonitrile) at a certain speed, and reversely flushed the slide rail pipeline, organic filter head, and stainless steel needle to the waste liquid. The mechanical turntable rotated to the organic solvent storage bottle, and the slide rail went down to puncture the organic solvent storage bottle and stayed for a period of time. The third syringe pump positively sucked a same volume of the organic solvent, and the solenoid valve communicated with the high-pressure flow path switching valve. The third syringe pump pumped the organic solvent to forwardly clean the pipeline to the waste liquid bottle connected to the high-pressure flow switching valve. According to the above process, backwashing and forward washing each were repeated once more, and the back and forward washing twice in total made the pipeline substantially clean. The slide rail went up and turned back to the position of the reactor 1 for the next sampling and sample injection. Control parameters: the H1 was the sampling time (s), the H2 was the sampling position, the H3 was the sampling speed (µL/s), the H4 was the sampling capacity, and the H5 was the dilution time (s).

So far, the hardware and control scheme of the online monitoring system could run stably according to the designed logic relationship. Regardless of whether the "monitoring result" instruction was used or not, after the "sampling and monitoring" instruction finished running, HPLC could give a near real-time status of the current reaction solution. This facilitated the optimization and regulation of subsequent or next automatic preparation of the same target molecule.

14. The operation of the "monitoring result" instruction was that: on the "online monitoring" interface of the upper computer, the logical relationship of the data report was set to record, compare, feedback, and control whether the automatic operation continued. Generally, the "monitoring result" was executed next to the "sampling and monitoring", and of course it could also be operated separately under manual control. The main function of this instruction was to extract a latest TXT report, and compare and determine it with a previous TXT report. If a result met expectations, automation was continued. If the result exceeded the set limit, an alarm was given to prompt the reason for the limit and wait for human processing. In this way, the user could choose to continue or stop the automatic operation according to the actual situations.

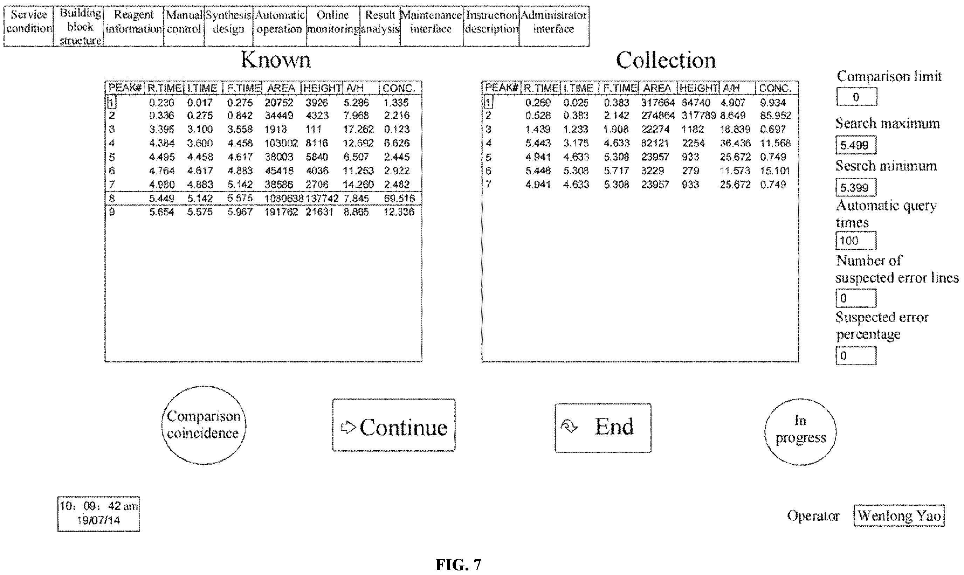

During the automatic operation, when the "sampling and monitoring" instruction was run, the "monitoring result" interface extracted the latest TXT report in the database folder in advance as known data (FIG. 7), and kept refreshing the folder to wait for an updated TXT Report. When the "sampling and monitoring" instruction was finished, the upper computer jumped from the "automatic operation" interface to the "monitoring result" interface. If the HPLC operation ended at this time and there was a new TXT report, the "monitoring result" interface could automatically and directly capture the latest data report as collected data (FIG. 7). If the HPLC did not finished running, the "monitoring result" interface continued to refresh the data folder and waited for the latest data report, and capture it as the collected data (FIG. 7).

For the convenience of comparison and determination, not all the contents of the original data output by HPLC were extracted, but the most important and most commonly-used parameters were extracted in order of the original data (FIG. 7). These parameters included: PEAK (number of peaks), R.TIME (retention time), AREA (peak area), HEIGHT (peak height), A/H (area/height), and CONC. (concentration ratio). The standard of comparison was the retention time and concentration ratio. Because different compounds had different retention times under a same analytical method, while the same compound had basically the same retention time under the same analytical method.

A specific logical relationship of designing the "online monitoring" interface was based on the normal "pre-activation" of one-pot automatic preparation (FIG. 4). After extracting the known data, the size of CONC. was compared and determined. A maximum value in the CONC. was selected as a concentration ratio of an unactivated "donor" or a newly-added "acceptor" after activation, and this data line was highlighted. Because both the donor and the acceptor had a strong UV-absorbing glucosinolate leaving group. In normal one-pot glycosylation, by setting a starting time of monitoring, the strong ultraviolet by-products with extremely small polarity generated after the activation of glucosinolate by the activator were excluded, and the compounds with strong ultraviolet absorption were only donors, acceptors, and possible new products. After extracting the maximum value of CONC., the corresponding R.TIME was found, and the search range in the collected data was expanded by the corresponding R.TIME ±0.05 (variable), to avoid the small difference of retention time of the same compound under the same analysis method. In the collected data, the value after the known data R.TIME±0.05 was searched for the second comparison and determination. If no corresponding data was found, it meant that the current state of the reaction solution had no "donor" or "acceptor", indicating that the "donor" or "acceptor" had been fully activated or completely reacted and disappeared. If the same R.TIME as in the known data was still found in the collected data, it meant that the current state of the reaction solution was that there were still "donors" and "acceptors" that had not been activated or had not completely reacted, then the corresponding CONC. was found. At this point, a "comparison limit" parameter of CONC. was designed (filled in according to experience and specific experimental requirements during writing the automation program) to continue the third comparison and determination: if the CONC. in the searched collected data was lower than the set "comparison limit" value, it meant that although there were still unactivated or incompletely reacted "donors" and "acceptors", the concentration ratio was highly low. This was in line with the expected reaction process, and the automatic preparation device was continued to operate. If CONC. was higher than the set "comparison limit" value, it meant that the remaining amount of "donor" and "acceptor" that were not activated or not fully reacted exceeded the expectations. At this time, the upper computer gave an alarm prompt and highlighted this data line, and the automation paused and waited for the user processing: if the user processing selected continuing, the subsequent automation continued. When there were "sampling and monitoring" and "monitoring result" instructions running, the above logical determination was repeated until the entire automatic preparation device completed its work, and a report of the automatic preparation device was generated. If the user processing selected stopping, all subsequent program instructions were terminated. This also generated an automatic preparation device report, which only showed the building block information prior to the stopping.

Through the three progressive automatic comparisons and determinations of the "online monitoring" interface of the upper computer, the reaction process could be clearly monitored and tracked. If there was an alarm prompt for the remaining "receptor", the user could also make the most reasonable treatment according to the near real-time reaction status.

Of course, the "sampling and monitoring" and "monitoring result" instructions were optional, which could or could not be used.

Post-automation: automatic synthesis results were saved, summarized, and analyzed. After the reactor was disassembled, the reaction solution was filtered with diatomaceous earth, and an obtained filtrate was concentrated under reduced pressure and separated by column chromatography and preparative liquid phase to obtain a white solid compound DEFGH-2, and the reactor was cleaned and dried for later use. The building blocks and reagents from storage bottles were recycled and washed. The used building blocks and reagent solutions were replaced with dichloromethane, and a "sample bottle cleaning" instruction was set (parameters: switching valve channel number, flow rate (µL/s), injection volume (µL)), so as to automatically clean the storage bottles that had stored samples in sequence for the next automation. Finally, the "gas protection" instruction was executed to release the gas pressure in the pipeline, the software was stopped and then the synthesizer was turned off. The automation differences of different target compounds were mainly related to the activation system, activation temperature, reaction temperature, and reaction time, and the overall automation actions were basically the same.

In the present disclosure, the automatic on-line monitoring was based on a self-developed synthesizer equipped with automatic sampling and injection equipment. After the automatic sampling and injection were completed, the software of the upper computer automatically gave a short-circuit signal to trigger the HPLC (Shimadzu LC-20A Analytical HPLC) to run in batches and sequentially under the instruction control. A specific analysis method included: 60% B to 100% B gradient elution was conducted for 8.0 min with the Shimadzu LC-20A analytical HPLC, and monitored by a UV variable-wavelength detector (254 nm and 280 nm), where a chromatographic column was: WatersC18, 5 µm, 2.1 mm*50 mm, and a mobile phase included: an A phase: H₂O+0.037% (v/v) TFA and a B phase: ACN+0.018% (v/v) TFA.

An automatic preparation method of a fondaparinux sodium pentosaccharide intermediate DEFGH-2 (Methyl 6-*O*-acetyl-3,4-di-*O*-benzyl-2-deoxy-2-azido-α-D-glucopyranosyl-(1→4)-(Methyl 2.3-di-*O*-benzyl-β-D-glucopyranosyluronate)-(1→4)-3,6-di-*O*-acetyl-2-deoxy-2-azido-α-D-gluc opyranosyl-(1→4)-(methyl 2-*O*-acetyl-3-*O*-benzyl-a-L-idopyranosyluronate)-(1→4)-6-*O*-benzoyl-3-*O*-benzyl-2-deoxy-2-be nzyloxycarbonylamino-α-D-glucopyranoside) included the following steps:

The compound D-3 (49.80 mg, 0.096 mmol), the compound EF-4 (60.1 mg, 0.08 mmol), and the compound GH-2 (53.97 mg, 0.064 mmol) were prepared. According to a general automation procedure of a *p*-TolSCl (770 µL taken from 90 µL diluted to 5000 µL)/AgOTf [2500 µL taken from 246.8 mg dissolved in 10000 µL of a mixed solution (8 mL toluene + 2 mL DCM)] activation system, column chromatography separation (petroleum ether/ethyl acetate, 2:1) and preparative liquid-phase separation were conducted to obtain a white compound 3-81 (73 mg, 0.03840 mmol, 60%). At present, the preparation method was implemented by the automatic preparation device, and a synthesis amount of the pentosaccharide DEFGH-2 had reached the gram scale, and it was expected to continue to optimize the efficiency and scale up. FIG. 10 showed a schematic diagram of the reaction of the compound D-3, the compound EF-4, and the compound GH-2 to generate DEFGH-2. The characterization result of gained compound DEFGH-2 was: R*_{f}*=0.12(petroleumether/ethylacetate, 2:1); ¹HNMR(600MHz, CDCl₃)δ8.11(d, *J*=7.3Hz, 2H), 7.57(t, *J*=7.4Hz, 1H), 7.46(t, *J*=7.7Hz, 2H), 7.40-7.30(m, 30H), 7.26-7.20(m, 5H), 5.55(d, *J*=3.8Hz, 1H), 5.48(d, *J*=4.0Hz, 1H), 5.40-5.35(m, 1H), 5.07-5.01(m, 5H), 4.90(d, *J*=5.9Hz, 2H), 4.87(d, *J*=6.5Hz, 1H), 4.86(s, 1H), 4.85-4.83(m, 2H), 4.82(s, 1H), 4.80(dd, *J*=5.1, 2.8Hz, 2H), 4.77(d, *J*=5.5Hz, 2H), 4.71(d, *J*=10.9Hz, 2H), 4.59(d, *J*=11.0Hz, 2H), 4.48(d, *J*=11.8Hz, 1H), 4.43-4.37(m, 2H), 4.31(d, *J*=11.5Hz, 1H), 4.25-4.18(m, 2H), 4.09(dd, *J*=7.1, 4.7Hz, 2H), 4.06-4.01(m, 3H), 3.96-3.93(m, 1H), 3.91-3.87(m, 2H), 3.79(s, 3H), 3.74(dt, *J*=15.4, 9.4Hz, 2H), 3.63(t, *J*=9.7Hz, 1H), 3.56(s, 3H), 3.54(d, *J*=6.3Hz, 2H), 3.46(dd, *J*=8.9, 8.1Hz, 1H), 3.37(s, 3H), 3.31(dd, *J*=10.4, 3.7Hz, 1H), 3.22(dd, *J*=10.8, 3.4Hz, 1H), 2.10(s, 3H), 2.09(s, 3H), 2.07(s, 3H), 2.04(s, 3H).¹³CNMR(151MHz, CDCl₃)δ170.66(Ac, C=O), 170.11(x2, Ac, C=O), 170.03(Ac, C=O), 169.51(COOMe, C=O), 168.39(COOMe, C=O), 166.16(Bz, C=O), 155.85(Cbz, C=O), 138.29, 138.10, 137.57, 137.56, 137.49, 137.33, 136.30, 133.11, 129.86, 128.61, 128.56, 128.55, 128.52, 128.50, 128.44, 128.39, 128.28, 128.18, 128.09, 128.07, 128.05, 128.03, 127.99, 127.94, 127.91, 127.64, 127.58, 127.35, 127.24, 127.07, 103.28(C-1), 98.77(C-1), 98.09(C-1), 97.62(C-1), 97.57(C-1), 83.78, 81.64, 80.14, 78.81, 77.03, 76.28, 75.79, 75.54, 75.22, 75.17, 74.99, 74.70, 74.42, 73.69, 73.25, 69.71, 69.61, 69.42, 69.34, 66.96, 63.26, 62.73, 62.22, 61.40, 60.87, 55.28, 54.60, 52.68, 52.22, 20.88, 20.86, 20.76, 20.69.HRMS(ESI⁺)Anal.CalcdforC₉₈H₁₀₇N₇O₃₂Na [M+Na]⁺, 1916.6853, found1916.6826.

Each example of the present specification is described in a progressive manner, each example focuses on the difference from other examples, and the same and similar parts between the examples may refer to each other.

Specific examples are used herein to explain the principles and embodiments of the present disclosure. The foregoing description of the embodiments is merely intended to help understand the method of the present disclosure and its core ideas; besides, various modifications may be made by those of ordinary skill in the art to specific embodiments and the scope of application in accordance with the ideas of the present disclosure. In conclusion, the content of the present specification shall not be construed as limitations to the present disclosure.

## Claims

1. An automatic preparation method of a fondaparinux sodium pentosaccharide intermediate, wherein the automatic preparation method is implemented by an automatic preparation device of the fondaparinux sodium pentosaccharide intermediate; the automatic preparation device comprises: a reactor, an upper computer, an inert gas device, a first activator container, a second activator container, a first sample container, a second sample container, a third sample container, an automatic sample injection system, a low-temperature circulating device, and a magnetic stirring device;
the inert gas device is connected with the automatic sample injection system, the first activator container, the second activator container, the first sample container, the second sample container, and the third sample container respectively; the automatic sample injection system is connected with the reactor; and the automatic sample injection system, the low-temperature circulating device, and the magnetic stirring device each are connected with the upper computer;
thefondaparinux sodium pentosaccharide intermediate has a structure shown in formula I, and is named DEFGH-1; 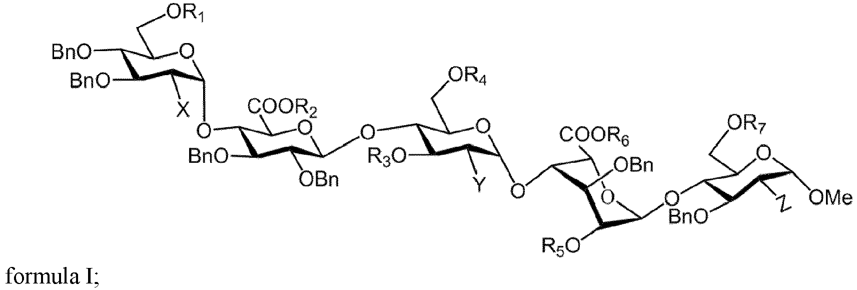
in the formula I, R₁ is selected from a group consisting of an acyl groupand a silicon protecting group; R₂ and R₆ are independently carboxyl protecting groups; R₃ and R₄ are independently acyl groups; R₅ and R₇ are independently acyl groups; and X, Y, and Z are independently selected from the group consisting of N₃ and various amino groups with a protecting group;
thefondaparinux sodium pentosaccharide intermediate is prepared from a compound D-1, a compound EF-1, and a compound GH-1; and
the automatic preparation method comprises the following steps:
adding the compound D-1 into the first sample container; wherein the compound D-1 has a structure shown in formula II: 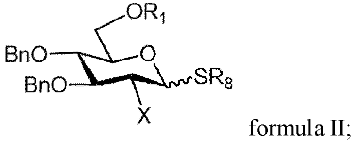
in the formula II, R₁ is selected from the group consisting of an acyl group and a silicon protecting group; SRs is a glucosinolate leaving group; and X is selected from the group consisting of N₃ and various amino groups with a protecting group;
adding the compound EF-1 into the second sample container; wherein the compound EF-1 has a structure shown in formula III: 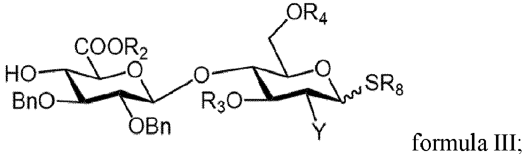
in the formula III, R₂ is a carboxyl protecting group, and R₃ and R₄ are independently acyl groups; SRs is a glucosinolate leaving group; and Y is selected from the group consisting of N₃ and various amino groups with a protecting group;
adding the compound GH-1 into the third sample container; wherein the compound GH-1 has a structure shown in formula IV: 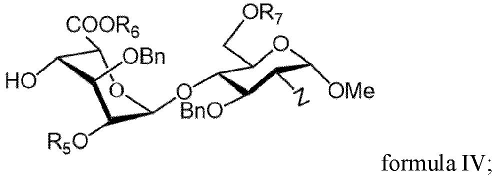
in the formula IV, R₅ and R₇ are independently acyl groups, R₆ is a carboxyl protecting group, and Z is selected from the group consisting of N₃ and various amino groups with a protecting group;
gas protection: filling the first sample container, the second sample container, the third sample container, the first activator container, the second activator container, and the reactor with an inert gas by the automatic sample injection system;
cooling the reactor by the low-temperature circulating device;
delivering the compound D-1 to the reactor by the automatic sample injection system;
stirring the compound D-1 in the reactor by the magnetic stirring device, and conducting pre-drying;
delivering a first activator in the first activator container and a second activator in the second activator container to the reactor sequentially by the automatic sample injection system, and conducting pre-activation I on the compound D-1 for a first set time;
delivering the compound EF-1 to the reactor by the automatic sample inj ection system;
subjecting the reactor to programmed heating by the low-temperature circulating device, and conducting a reaction I for a second set time;
cooling the reactor by the low-temperature circulating device;
delivering the first activator in the first activator container and the second activator in the second activator container to the reactor sequentially by the automatic sample injection system, and conducting pre-activation II on an intermediate obtained by a reaction of the compound D-1 and the compound EF-1 for a third set time;
delivering the compound GH-1 to the reactor by the automatic sample injection system;
subjecting the reactor to programmed heating by the low-temperature circulating device, and conducting a reaction II for a fourth set time; and
delivering a quenching reaction solvent to the reactor by the automatic sample injection system, and terminating the reaction II to obtain a compound DEFGH-1.

2. The automatic preparation method of a fondaparinux sodium pentosaccharide intermediate according to claim 1, wherein the first activator is p-Toluene sulfonyl chloride (p-TolSCl), and the second activator is silver trifluoromethanesulfonate (AgOTf).

3. The automatic preparation method of a fondaparinux sodium pentosaccharide intermediate according to claim 1, wherein the reactor is cooled to -75°C by the low-temperature circulating device.

4. The automatic preparation method of a fondaparinux sodium pentosaccharide intermediate according to claim 1, wherein the magnetic stirring device has a rotational speed of 400 rpm to 1,000 rpm.

5. The automatic preparation method of a fondaparinux sodium pentosaccharide intermediate according to claim 1, wherein the compound D-1 in the reactor is stirred for 1 min to 300 min by the magnetic stirring device.

6. The automatic preparation method of a fondaparinux sodium pentosaccharide intermediate according to claim 1, wherein the automatic preparation device further comprises a detection device; and the detection device is connected to the reactor by means of a pipe, and is electrically connected to the upper computer.

7. The automatic preparation method of a fondaparinux sodium pentosaccharide intermediate according to claim 6, further comprising the following steps after the pre-activation I is conducted on the compound D-1 for the first set time:
detecting whether there is remaining compound D-1 by the detection device, and conducting alarming if there is the remaining compound D-1.

8. The automatic preparation method of a fondaparinux sodium pentosaccharide intermediate according to claim 6, further comprising the following steps after the pre-activation II is conducted on the intermediate obtained by the reaction of the compound D-1 and the compound EF-1 for the third set time:
detecting whether there is remaining intermediate obtained by the reaction of the compound D-1 and the compound EF-1 by the detection device, and conducting alarming if there is the remaining intermediate.

9. The automatic preparation method of a fondaparinux sodium pentosaccharide intermediate according to claim 6, further comprising the following steps after the reactor is subjected to the programmed heating by the low-temperature circulating device, and the reaction II is conducted for the fourth set time:
detecting whether there is remaining compound GH-1 by the detection device, and conducting alarming if there is the remaining compound GH-1.

10. The automatic preparation method of a fondaparinux sodium pentosaccharide intermediate according to claim 1, wherein the quenching reaction solvent is triethylamine.
